# EUROPEAN PATENT APPLICATION

(11) **EP 4 840 456 A1**
(43) Date of publication of application: **18.11.2026**
(21) Application number: 25176508.7
(22) Date of filing: 14.05.2025
(51) Int. Cl.: A61K 36/48, A61K 35/741, A23L 33/00, A61P 25/28

(54) **TAMARIND COMPOSITIONS AND USES THEREOF**

(71) Applicant: Trilliome GmbH, 8049 Zürich (CH)
(72) Inventor: Mak, Tim Nam, Zürich 8050 (CH); Guye-Vuillème, Patrick, 8049 Zürich (CH)
(74) Representative: Mewburn Ellis LLP

(57) **Abstract**

The invention relates to compositions comprising extracts from tamarind plant substrates, their use in enhancing beneficial bacteria and metabolite abundance, and methods of obtaining such compositions. The invention further encompasses pharmaceutical compositions comprising tamarind extracts and their use in enhancing abundance of bacterial species known to enhance cognitive function.

## Description

### Field

The present invention relates to methods of obtaining extracts of tamarind fruit pulp and tamarind seed, and uses including enhancing the abundance of beneficial bacteria in the intestinal tract, alone, or in combination with probiotic compositions.

### Background

The human gut microbiome plays a critical role in maintaining cognitive health, metabolic homeostasis, and systemic well-being through its influence on neurotransmitter synthesis, hormone regulation, and immune signaling. The abundance of specific beneficial bacterial taxa within this complex ecosystem has been directly correlated with reduced neuroinflammation, improved age-related cognitive function, and enhanced overall physiological resilience. Despite the recognized therapeutic potential of modulating gut microbiota, existing strategies-such as prebiotics, probiotics, and synbiotic combinations-are limited by inherent challenges that hinder their efficacy, specificity, and applicability to modern health demands.

Probiotic interventions, which aim to introduce beneficial commensal bacteria into the gastrointestinal tract, face significant barriers. Many key taxa, such as obligate anaerobes, are refractory to culturing in laboratory settings, complicating large-scale production and delivery. Furthermore, post-administration engraftment of probiotic strains is often inconsistent due to competition from endogenous microbes, niche saturation within the gut environment, and suboptimal survival under gastrointestinal conditions. These limitations reduce their reliability as a means to sustainably enhance beneficial bacterial populations.

Prebiotics-compounds that selectively stimulate growth or activity of health-promoting bacteria-are an alternative strategy. While they offer advantages such as targeted modulation of native microbiota in situ, current prebiotic formulations derived from plant sources (e.g., fibers and polyphenols) face practical constraints. Raw plant materials may require impractical consumption volumes to achieve therapeutic benefits, while excessive intake can lead to undesirable side effects due to co-ingestion of compounds like phytates, oxalates, or excess sugars. For example, high quantities of sugary fruits or acidic foliage may negate health gains by contributing to metabolic disturbances or gastrointestinal discomfort.

Synbiotic formulations, which combine probiotics and prebiotics to synergistically enhance microbial survival and efficacy, represent a promising avenue. However, existing approaches often lack systematic optimization of the symbiotic relationship between specific probiotic strains and their corresponding growth substrates. This suboptimal pairing limits the potential to maximize probiotic engraftment and metabolic activity within target niches of the gut microbiome.

The tamarind (Tamarindus indica) tree, widely cultivated across tropical regions, possesses underutilized bioactive components in its seed, pulp, leaves, and other tissues. Despite anecdotal associations with health benefits (e.g., antimicrobial, anti-inflammatory, and antioxidant activities), certain parts of the plant-such as bitter leaves or hard seeds-are largely unsuitable for direct consumption due to sensory limitations. Furthermore, few studies systematically investigate the isolation, characterization, or microbiome-modulating potential of bioactive compounds like polysaccharides and polyphenols derived from distinct tamarind tissues. This gap precludes the development of targeted, plant-based interventions that circumvent the drawbacks of raw material consumption while harnessing the therapeutic potential of these compounds.

In light of these unresolved challenges, there remains a critical need for stabilized, precisely engineered prebiotic compositions and synbiotic formulations capable of: (i) selectively enhancing beneficial commensal bacteria-both probiotic strains and endogenous taxa associated with cognitive and metabolic health; (ii) overcoming limitations in scalability, engraftment efficiency, and consumer acceptability inherent to current microbiome-modulating approaches; and (iii) leveraging underexplored plant sources like tamarind to produce bioactive extracts free of confounding compounds.

Based on the above-mentioned state of the art, the objective of the present invention is to provide means and methods to enhance the abundance of beneficial bacteria and metabolites in the intestine, particularly to increase the abundance of specific commensal bacteria delivered as probiotics, or endogenous commensal bacteria associated with significant benefits. This objective is attained by the subject-matter of the independent claims of the present specification, with further advantageous embodiments described in the dependent claims, examples, figures and general description of this specification.

### Summary of the Invention

The present invention introduces novel compositions and methods for modulating the human gut microbiome using tamarind extracts. The independent claims recite the use of tamarind seed extract (SE), full fruit pulp extract, and/or polysaccharide, or polyphenol-rich fractions of tamarind fruit pulp extract (FE/PFS/PFF) as active agents capable of altering microbial metabolism and composition. The extracts may be administered alone as pre-biotics or postbiotics, or in combination with live bacteria preparation in probiotics, to enhance beneficial bacterial species' abundance in the intestine, and metabolite production associated with anti-inflammatory properties and cognitive health benefits.

Using an in-vitro model system based on donor stool samples, the inventors demonstrate that tamarind extracts significantly alter gut bacterial metabolite production and microbial composition. These changes correlated with specific beneficial bacteria stimulation, including *Bifidobacterium adolescentis*/*longum, Blautia spp., Coprococcus catus, Anaostipes, Agathobaculum butyriciproducens, Oliverpens,* and *Parabacteroides distasonis,* which are known to have positive effects on cognitive function. The SE fraction strongly stimulates bacteria with known modulatory effects on neuroinflammatory processes, such as *Agathobaculum butyriciproducens* and *Coprococcus catus.*

The invention relates to use of these compositions in increasing the relative abundance of beneficial bacterial or short chain fatty acid metabolites in the intestine of a subject, in improving symptoms of cognitive impairment in a patient, and/or in improving symptoms of a patient diagnosed with depression, Alzheimer's disease, Parkinson's disease, age-related cognitive impairment, or an autism spectrum disorder in a human subject.

Accordingly, in one aspect, the invention provides a composition comprising a tamarind fruit extract for use in increasing the relative abundance of a beneficial bacteria in the intestine of a patient; and/or in increasing the presence of acetate, propionate, and/or butyrate in the intestine of a patient. In some embodiments, the composition comprises a tamarind seed extract, and/or a tamarind fruit pulp extract.

In some embodiments, the beneficial bacteria is a species selected from *Collinsella aerofaciens, Anaerobutyricum hallii, Blautia faecis, Mediterraneibacter faecis, Oliverpabstia intestinalis, Coprococcus comes, Parabacteroides distasonis, Butyrate-producing bacterium SS3*/*4, Faecalibacterium prausnitzii, Fusicatenibacter saccharivorans, Lachnoclostridium pacaense, Coprococcus catus, Blautia fusiformis, Blautia obeum, Anaerostipes hadrus, Agathobaculum butyriciproducens, Faecalibacillus intestinalis, Fusicatenibacter saccarivorans, Bifidobacterium adolescentis, Bifidobacterium bifidum,* and *Bifidobacterium longum.*

In some embodiments, the composition comprising a tamarind fruit extract is a symbiotic preparation, further comprising a beneficial bacterial species. In particular embodiments, the live bacteria preparation in the symbiotic comprising a tamarind fruit extract is selected from *Collinsella aerofaciens, Anaerobutyricum hallii, Blautia faecis, Mediterraneibacter faecis, Oliverpabstia intestinalis, Coprococcus comes, Parabacteroides distasonis, Butyrate-producing bacterium SS3*/*4, Faecalibacterium prausnitzii, Fusicatenibacter saccharivorans, Lachnoclostridium pacaense, Coprococcus catus, Blautia fusiformis, Blautia obeum, Anaerostipes hadrus, Agathobaculum butyriciproducens, Faecalibacillus intestinalis, Fusicatenibacter saccarivorans* and a species in the genus *Bifidobacterium.* In preferred embodiments, the symbiotic composition comprises a species selected from *Bifidobacterium adolescentis, Bifidobacterium bifidum,* and *Bifidobacterium longum.* In further preferred embodiments, the symbiotic composition comprises the SR79 strain with Accession No. KCTC 13036BP.

The composition may be for use in improving symptoms of cognitive impairment in a patient suffering therefrom, particularly wherein the composition comprises a tamarind seed extract, and/or a tamarind fruit pulp extract. These improvements may be associated with the increased relative abundance of a beneficial bacteria in the intestine of the patient; and/or increased acetate, propionate, and/or butyrate levels in the intestine of the patient.

The composition may be for use in the treatment of a disease or disorder that causes or is associated with neuroinflammation. The patient may be suffering from or be diagnosed with depression, Alzheimer's disease, Parkinson's disease, age-related cognitive impairment, or an autism spectrum disorder and the therapeutic use may improve the respective disease or symptoms. The therapeutic use of the compositions of the invention may improve these conditions by increasing the relative abundance of a beneficial bacteria in the intestine of the patient; and/or in increasing the presence of acetate, propionate, and/or butyrate in the intestine of the patient.

In another aspect, the invention provides a non-therapeutic method of improving the cognition, mood and/or quality of life (QoL) in a subject as measured using a clinical scoring method, wherein the method comprises orally administering a composition comprising a tamarind fruit extract to the subject. In some embodiments, the composition comprises a tamarind seed extract, and/or a tamarind fruit pulp extract as defined herein. The clinical scoring method may be EQ-5D-5L, FACIT-F or MSE. The subject may be a healthy and/or elderly individual. In some embodiments, the subject is at least 55, at least 60, at least 65, at least 70, at least 75 or at least 80 years old. The mechanism and beneficial bacteria associated with these non-therapeutic methods are as described herein.

The invention further relates methods of preparing tamarind compositions from tamarind plant matter (particularly tamarind fruit pulp, or tamarind seed) according to the invention, methods of manufacturing foods or drinks supplemented with said extracts, as well as tamarind fruit preparations obtainable by these processes.

### Terms and definitions

### General

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term tamarind plant refers to the plant species *Tamarindus indica. Tamarindus indica* belongs to the family *Fabaceae,* and the genus *Tamarindus* is monotypic, meaning it contains this single species having cultivars referred to as sweet tamarind and sour tamarind. The tamarind fruit comprises seeds, and a fruit pulp surrounding the seeds. The tamarind seed comprises a shell, known as a *testa,* and a central kernel.

The term *relative abundance* of beneficial bacteria is a measure used to reflect microbiome composition in a sample obtained from a biological niche. Determination of relative abundance can be obtained, for example for a faecal sample obtained from a human subject as measure of intestinal bacterial diversity. An operational taxonomic unit or OTU is a unit reflecting microbial diversity often employed to sequencing of small subunit 16s sequences from prokaryotes to delineate species-level distinctions. An OTU clustering algorithm applied to sequencing output of a nucleic acid sample can thus provide a value reflecting the relative abundance of a species, with reference to the absolute number of microorganisms observed. This measure can be applied to samples obtained prior to, and following administration of an intervention such as a composition comprising tamarind according to the invention to confirm any change in abundance. Although OTUs can be calculated differently using different algorithms or thresholds (such as the Bayesian method applied herein), OTU are generally consistent across habitats and several OTU clustering approaches.

The terms *patient* and *subject* are used herein interchangeably. The subject to be treated may be any animal, e.g. human. The subject is preferably mammalian, more preferably human. The subject may be a non-human mammal, but is more preferably human. The subject may be male or female. The subject may be a patient suffering from and/or diagnosed with a disease. Therapeutic uses may be in human or animals (veterinary use).

As used herein, the term *treating* or *treatment* of any disease or disorder (e.g. cancer) refers in one embodiment to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Organic Chemistry

The term *organic solvent* in the context of the present invention relates to volatile carbon-based chemicals capable of dissolving or dispersing one or more other chemical substances. Specific examples include alkanes, such as hexane, or n-hexane, acetone, and ethyl acetate.

The term *alkane* in the context of the present specification relates to a saturated hydrocarbon comprising only carbon and hydrogen atoms, lacking double or triple bonds. Alkanes of use according to the invention are liquids which may be used as a non-polar solvent. The term n-*hexane* refers to a straight-chain alkane with six carbon atoms and the molecular formula C₆H₁₄.

The term *alcohol* in the context of the present specification relates to a series of hydroxyl compounds having the general formula CₙH₂ₙ₊₁OH. Particular examples include ethanol and methanol.

The term *alkane* in the context of the present specification relates to a series of non-aromatic saturated acyclic hydrocarbons with the general formula CₙH₂ₙ₊₂. Particular examples include hexane and n-hexane. Hexane is a mixture of branched and unbranched molecules having the chemical formula C₆H₁₄, and n-hexane is the linear (unbranched) form of hexane.

The term *polyphenol compound* in the context of the present specification relates to natural small compounds derived from tamarind plant matter with a molecular weight of in the range of 500-4000 Da and characterised by phenolic hydroxyl groups and/or aromatic rings.

A *monosaccharide* is a sugar comprising three to seven carbon atoms. Examples are glyceraldehyde (C3), erythrose or threose (C4), arabinose, ribose or xylose (C5) glucose, mannose, galactose or fructose (C6) or sedoheptulose (C7). The sugar alcohols and amino sugars of C3 to C7 monosaccharides are included in the group of monosaccharides according to the definition used herein. An *oligosaccharide* is a molecule consisting of two to ten of the same or different monosaccharides according to the above definition. A *polysaccharide* comprises more than ten monosaccharides.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavouring agents, dyes, and the like and combinations thereof, as would be known to those skilled in the art (see, for example, Remington: the Science and Practice of Pharmacy, ISBN 0857110624). The invention also encompasses nanoparticles, liposomes, or cellular carriers within the meaning of *pharmaceutically acceptable carrier.*

### Quality of life and clinical scoring methods

Quality of life (QoL) relates to a patient's sense of well-being and ability to carry out activities of daily living. Several methodologies are known in the art and may be used to assess QoL in subjects, e.g. in patients suffering from neuroinflammatory diseases. A clinical test of general health status is EQ-5D-5L. A clinical test of patient fatigue is FACIT-F. A clinical test of cognitive function, behaviour and mood is the MSE.

EQ-5D-5L is a standardised measure of health status which provides a simple, generic measure of health for clinical and economic appraisal (The EuroQol Group, 1990) and may be carried out as described in the EQ-5D-5L User Guide, version 2 (Oemar and Janssen, 2013) which is hereby incorporated by reference in its entirety. The EQ-5D-5L consists of two components, a descriptive system and a visual analogue scale (EQ VAS). The descriptive component comprises 5 "dimensions" to be measured (mobility, self-care, usual activities, pain/discomfort, anxiety/depression), with each dimension being scored on a 5-level scale (1 = no problem, 2 = slight problems, 3 = moderate problems, 4 = severe problems, 5 = extreme problems). Following treatment with a composition of the invention, one or more of the EQ-5D-5L dimensions (mobility, self-care, usual activities, pain/discomfort, and anxiety/depression) may be decreased by 1, 2, 3, or 4 points compared to the score prior to treatment.

The "functional assessment of chronic illness therapy - fatigue" (FACIT-F) is a measure that assesses self-reported patient fatigue and the impact said fatigue has on a patient's ability to perform daily activities and functions and can be performed as disclosed in Hewlett et al (2011), which is hereby incorporated by reference in its entirety. Originally developed for assessment of fatigue in cancer and anaemia patients, it has since become a standard test for assessment of fatigue in various diseases and disorders. Physical fatigue, functional fatigue, emotional fatigue and social consequences of fatigue are measured. 13 measures are taken to evaluate a subject's level of fatigue during their usual daily activities over the past week and reported on a Likert scale from 4 (not at all fatigued) to 0 (very much fatigued). Where measures are taken as negatively stated items, the scores are reversed by subtracting the response from 4. The scores for each of the 13 measures may then be summed to derive an overall score. A subject scoring less than 20 in the FACIT-F measure is considered to have severe fatigue. An increase of 4 points in FACIT-F score is the minimal clinically important difference (MCID). An increase of 9.7 points in FACIT-F score is the within-patient meaningful improvement (WPMI). Following treatment with a composition of the invention, a subject may have an increased FACIT-F score compared to their FACIT-F score prior to treatment, e.g. an MCID or a WPMI.

The mental status examination (MSE) is a structured assessment of the patient's mood, behaviour and cognitive functioning. It includes descriptions of the patient's appearance and general behaviour, level of consciousness and attentiveness, motor and speech activity, mood and affect, thought and perception, attitude and insight, the reaction evoked in the examiner, and, finally, higher cognitive abilities. The specific cognitive functions of alertness, language, memory, constructional ability and abstract reasoning are the most clinically relevant (Walker *et al,* 1990). Following treatment with a composition of the invention, a subject may have improved mood, behavioural and/or cognitive functioning as assessed by an MSE compared to their mood, behavioural and/or cognitive functioning as assessed by an MSE prior to treatment.

### Detailed Description of the Invention

A first aspect of the invention provides composition comprising a tamarind fruit extract for use in increasing in a recipient the relative abundance of beneficial bacteria in the intestine; and/or increasing the presence of acetate, propionate, and/or butyrate in the intestine.

In some embodiments the composition comprises, or consists of a tamarind seed extract, or a tamarind fruit pulp extract. In some embodiments, the composition for use in increasing beneficial bacteria or their metabolites comprises a combination of both tamarind fruit pulp extract, and a tamarind seed extract.

Another aspect of the invention relates to a tamarind fruit extract or use in improving symptoms of cognitive impairment in a patient.

In some embodiments of the composition for use according to the invention, embodiments, the composition comprises, or consists of tamarind seed extract. In particular embodiments, the composition comprises, or consists of tamarind seed kernel extract.

In some embodiments of the composition according to the invention, the composition comprises, or consists of a fraction obtained from a tamarind fruit pulp extract comprising a tamarind fruit pulp polyphenolic fraction comprising at least 40% polysaccharide compounds, and at least 50% polyphenol compounds. In other embodiments, the composition comprises a fraction obtained from a tamarind fruit pulp extract, comprising at least 50% polysaccharide compounds. In other embodiments, the composition comprises an un-fractionated fruit pulp extract.

Compositions comprising tamarind fruit extracts according to the invention are demonstrated in the examples to be useful as prebiotics that increase the relative abundance of beneficial bacteria such as Bifidobacterium species (in terms of genetic measures the operational taxonomic unit, or OTU), as well as the abundance (as determined by metabolic measurements in biological samples such as faecal samples) of bacteria-derived metabolites associated with improved well-being such as acetate, propionate, and/or butyrate.

In some embodiments the composition comprising a tamarind fruit extract is provided as a prebiotic, meaning it provided to enrich the abundance of target endogenous species present, or likely to be present in the intestine of the recipient.

In some embodiments the composition comprising a tamarind fruit extract is provided as a synbiotic, meaning a combination of a prebiotic as above, and a probiotic live beneficial bacterial strain. The presence of the prebiotic tamarind fruit extract in the synbiotic is provided to enrich the engraftment, and abundance of target beneficial bacteria species.

In some embodiments the composition comprising a tamarind fruit extract is provided for use to a subject as a postbiotic, meaning it provided to enrich the abundance of target beneficial bacteria species present which has recently been administered to a subject, for example on the same day, or in the same week, as a live bacterial preparation of the target beneficial bacteria species.

In some embodiments, the composition comprising a tamarind fruit extract is provided for use in increasing the relative abundance of a beneficial bacterial species selected from *Collinsella aerofaciens, Anaerobutyricum hallii, Blautia faecis, Mediterraneibacter faecis, Oliverpabstia intestinalis, Coprococcus comes, Parabacteroides distasonis, Butyrate-producing bacterium SS3*/*4, Faecalibacterium prausnitzii, Fusicatenibacter saccharivorans, Lachnoclostridium pacaense, Coprococcus catus, Blautia fusiformis, Blautia obeum, Anaerostipes hadrus, Agathobaculum butyriciproducens, Faecalibacillus intestinalis, Fusicatenibacter saccarivorans* and a species in the genus *Bifidobacterium,* particularly a species selected from *Bifidobacterium adolescentis, Bifidobacterium bifidum,* and *Bifidobacterium longum.*

In particular embodiments, the beneficial bacterial is a species in the genus *Blautia,* particularly a species selected from *B. fusiformis, B. obeum,* and *B*. *faecis.*

In particular embodiments, the beneficial bacterial is a species in the genus *Bifidobacterium,* particularly a species selected from *Bifidobacterium adolescentis, Bifidobacterium bifidum,* and *Bifidobacterium longum.*

In particular embodiments, the *A. butyriciproducens* targeted by the tamarind prebiotic, or present in a synbiotic tamarind composition consists of the SR79 strain with Accession No. KCTC 13036BP.

In some embodiments, the composition for use as above consists of tamarind fruit extract obtained only from seed material as the tamarind plant matter starting material. In some embodiments, the composition comprises, or essentially consists of tamarind seed extract. In particular embodiments, this sees extract is provided for use in increasing the relative abundance in the intestine of the beneficial bacteria *A. butyriciproducens.* In some embodiments, the composition comprises, or essentially consists of tamarind seed extract, and is provided for use in increasing the relative abundance in the intestine of the beneficial bacteria *C. catus.*

The invention further encompasses use of a composition comprising a tamarind seed extract as a low-flatulence prebiotic composition.

In some embodiments, the invention provides a tamarind fruit extract for use in improving symptoms of cognitive impairment in a patient. In certain embodiments, the composition is provided for use in improving symptoms of depression, Alzheimer's disease, Parkinson's disease, age-related cognitive impairment, or an autism spectrum disorder. In particular embodiments, the composition for improving cognitive impairment comprises, or essentially consists of tamarind seed extract.

Another aspect of the invention relates to a symbiotic preparation, a composition comprising a tamarind fruit extract in combination with a target beneficial bacterial species. In particular embodiments, the live bacteria preparation in the symbiotic comprising a tamarind fruit extract is selected from *Collinsella aerofaciens, Anaerobutyricum hallii, Blautia faecis, Mediterraneibacter faecis, Oliverpabstia intestinalis, Coprococcus comes, Parabacteroides distasonis, Butyrate-producing bacterium SS3*/*4, Faecalibacterium prausnitzii, Fusicatenibacter saccharivorans, Lachnoclostridium pacaense, Coprococcus catus, Blautia fusiformis, Blautia obeum, Anaerostipes hadrus, Agathobaculum butyriciproducens, Faecalibacillus intestinalis, Fusicatenibacter saccarivorans* and a species in the genus *Bifidobacterium,* particularly a species selected from *Bifidobacterium adolescentis, Bifidobacterium bifidum,* and *Bifidobacterium longum.*

Certain embodiments of the invention relate to a dosage form for enteral administration, such as nasal, buccal, rectal, transdermal or oral administration. In addition, the compositions of the present invention can be made up in a solid form (including without limitation capsules, tablets, pills, granules, powders or suppositories), or in a liquid form (including without limitation solutions, suspensions or emulsions).

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of obtaining tamarind fruit extracts

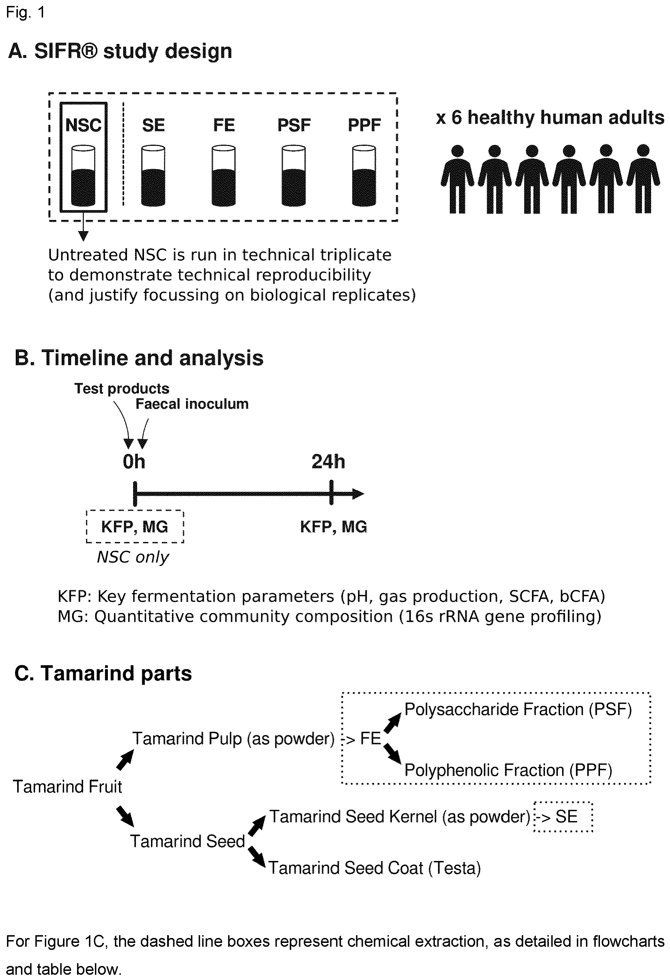

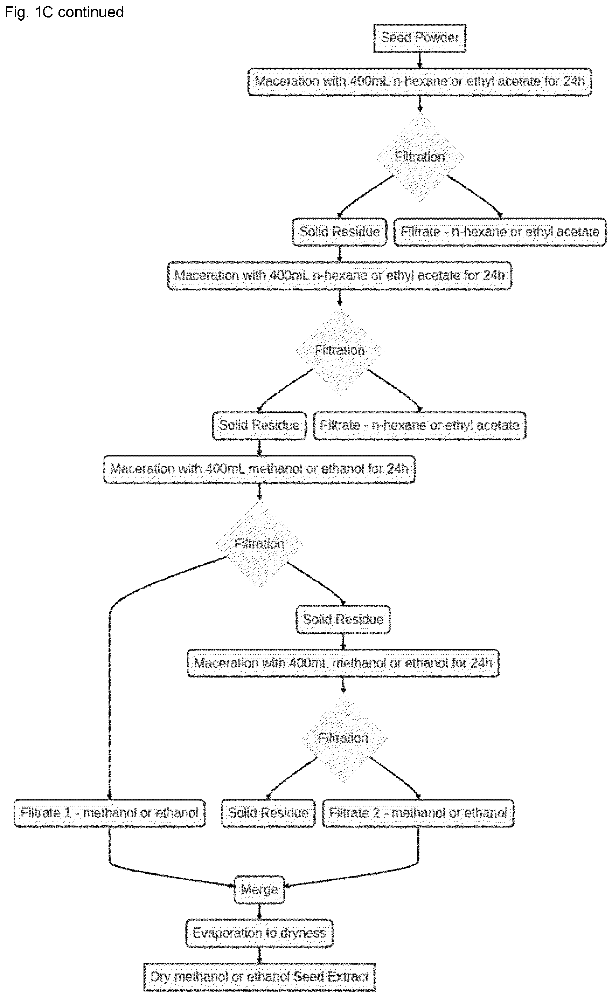

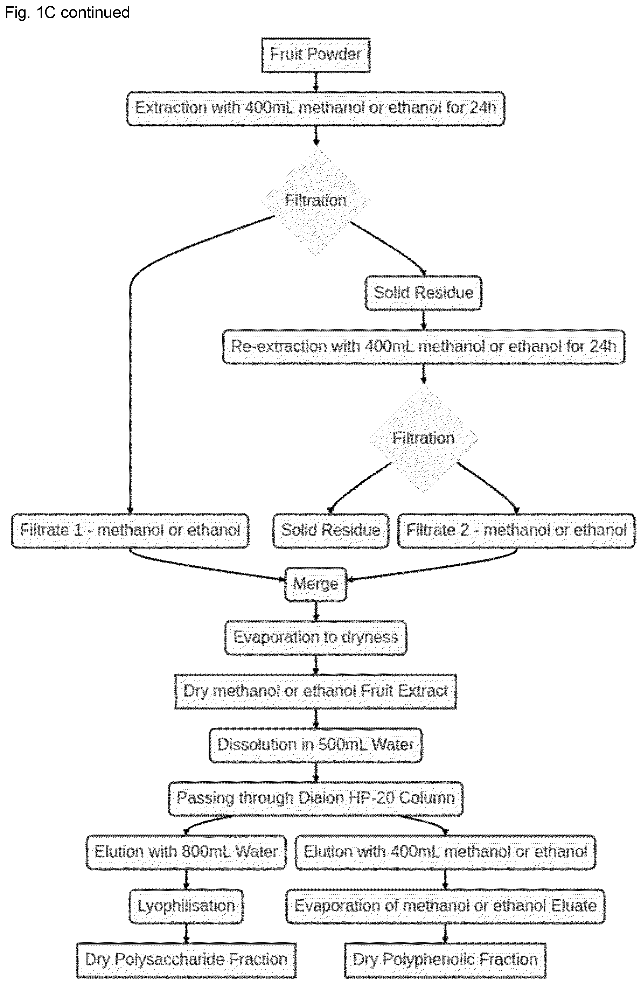

A further aspect of the invention is a method for obtaining a tamarind seed extract and/or a tamarind pulp extract comprising the following steps, which are also illustrated in the flowcharts in Figure 1C. The starting material is a tamarind plant matter selected from a tamarind seed preparation, or tamarind fruit pulp preparation, or a mixture of both. The tamarind plant matter can be raw fruit preparations obtained from seed, or seed kernel, or fruit pulp, or a mixture thereof. Alternatively, the starting material may be dried and powdered individual plant part preparations, or mixtures thereof.

Then in a solvent step, the tamarind plant matter is soaked, i.e. macerated in an alcohol, providing an alcoholic extract. After this, the next step is separating and removing the insoluble plant matter from the alcoholic extract, and subsequently removing the alcohol from the alcoholic extract to provide a dried tamarind seed extract, or a dried tamarind fruit pulp extract. In particular embodiments, the alcohol is removed by evaporation to provide the dried extract. In certain embodiments of the method, the tamarind plant matter is soaked, i.e. macerated in a solution of 100% methanol. In alternative embodiments, the tamarind plant matter is soaked in ethanol, preferably 100% ethanol.

Optionally, the method further comprises dissolving a dried tamarind fruit pulp extract obtained above in an aqueous solution (particularly water), and a further separation step to enrich polysaccharide compounds in the extract. In some embodiments, a fractionation step is applied to the re-hydrated dried extract, involving isolating from the aqueous solution comprising the dried tamarind fruit pulp extract a fraction comprising at least 40%, particularly at least 50% polysaccharide compounds. In particular embodiments the fractionation is a performed by chromatography.

In some embodiments, the method further comprises dissolving a dried tamarind fruit pulp extract obtained above in an aqueous solution (particularly water), and a further separation step to enrich both polysaccharide compounds and polyphenol compounds in the resulting extract. In some embodiments, a fractionation step is applied to the re-hydrated dried extract, involving isolating from the aqueous solution comprising the dried tamarind fruit pulp extract a fraction comprising at least 40% polysaccharide compounds, and at least 50% polyphenol compounds.

In particular embodiments, the fractionation step is performed by means of liquid chromatography (LC). In more particular embodiments, a fraction enriched in polysaccharide compounds, and/or polyphenol compounds is obtained by reversed-phase liquid chromatography (RPLC).

In some embodiments of the method as illustrated in Fig 1C with regard to a tamarind seed extract, prior to the alcohol soaking step, the method further comprises an organic solvent step. This involves soaking the tamarind plant matter with an organic solvent, for example, n-hexane, or ethyl acetate providing a solvent extract, then separating soluble residue from the solvent extract, and removing (discarding) the solvent from the solid residue. This solid residue is then used in the alcohol solvent step as specified Fig 1C, where the solid residue is soaked in the alcohol to provide the alcoholic extract as described above.

As organic solvent, the skilled person is aware alkanes or alkane mixtures (such as petroleum ether) would also be appropriate, whereas less lipophilic solvents may risk excluding from the extract some desired metabolites. In particular embodiments, the organic solvent is an alkane, or an alkane mixture. In more particular embodiments, the organic solvent is n-hexane.

In further particular embodiments, the organic solvent is ethyl acetate, which is widely used in preparation of food-safe items and pharmaceutical botanical extracts.

In some embodiments, the method comprises an alcohol solvent step using methanol, and an organic solvent step using n-hexane.

In some embodiments, the method comprises an alcohol solvent step using ethanol, and an organic solvent step using ethyl acetate.

In particular embodiments of the method according to this aspect of the invention, the duration of the soaking in the alcohol solvent step and/or the organic solvent step is at least 24 hours.

Further aspects of the invention relate to compositions obtainable by the methods described herein:
A composition comprising, or consisting of tamarind seed extract obtainable by the method according to the flow chart provided in Figure 1C.

A composition comprising, or consisting of tamarind fruit pulp extract obtainable by the method according to the flow chart provided in Figure 1C.

A composition comprising, or consisting of tamarind fruit pulp extract obtainable by the method according to the flow chart provided in Figure 1C containing at least 50% polysaccharide compounds.

A composition comprising, or consisting of tamarind fruit pulp extract obtainable by the method according to the flow chart provided in Figure 1C containing at least 50% polyphenol compounds and at least 40% polysaccharide compounds.

### Method of manufacture of prebiotic food or drink products

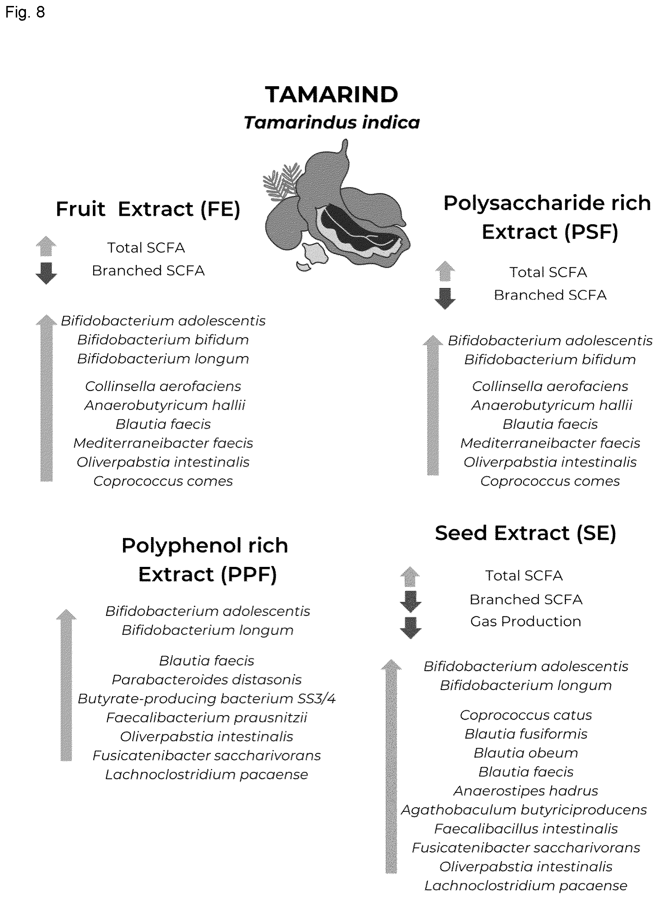

Another aspect of the invention relates to a method of making a food or drink product comprising a tamarind plant extract, providing a supplemented product with the capacity to increase the abundance of beneficial bacteria present in the subject's microbiome. The method comprises the steps of producing a tamarind seed, or seed kernel extract, or a fractionated fruit pulp extract as shown in Figure 1C. The tamarind fruit extract is then added to a food or drink preparation to provide a supplemented product. Tamarind extracts derived herein can be chosen to enhance the abundance of specific bacteria, or to reduce gas production depending on the extracts used, as summarised in Figure 8.

### Method of Manufacture and Method of Treatment according to the invention

The invention further encompasses, as an additional aspect, the use of a composition comprising a tamarind fruit extract as identified herein, in a prebiotic or probiotic format as specified in detail above, for use in a method of manufacture of a medicament for the treatment or prevention of a condition depression, Alzheimer's disease, Parkinson's disease, age-related cognitive impairment, or an autism spectrum disorder.

Similarly, the invention encompasses methods of treatment of disease, comprising administering to a patient in need thereof a therapeutically effective amount of tamarind fruit extract, in a prebiotic or probiotic format as specified in detail above.

Wherever alternatives for single separable features are laid out herein as "embodiments", it is to be understood that such alternatives may be combined freely to form discrete embodiments of the invention disclosed herein. Thus, any of the alternative embodiments for a feature 1 may be combined with any of the alternative embodiments of feature 2 and these combinations may be combined with any feature mentioned herein.

The invention is further illustrated by the following examples and figures, from which further embodiments and advantages can be drawn. These examples are meant to illustrate the invention but not to limit its scope.

### Description of the Figures

- Fig. 1: shows (A) study design SIFR, (B) timeline and analysis, (C) flow charts for obtaining botanical extracts from tamarind seed, and tamarind fruit, providing full seed extract (SE), full fruit extract (FE), fruit polysaccharide fraction (PSF), and fruit polysaccharide and polyphenol fraction (PPF).
- Fig. 2: shows the comparison of the effects of test products on gas production, total SCFA, acetate, propionate, butyrate, valerate, bCFA, isovalerate and isobutyrate against the no-substrate control (NSC). The effects are presented as % of increase (positive values) or decrease (negative values) from NSC (dashed line), as averaged across 6 donors. Standard deviation is indicated by vertical lines.
- Fig. 3: shows the impact of the test products on family level OTUs that were significantly (p adjusted < 0.20, indicated by *) or consistently affected (indicated by $) by any of the treatments, expressed as log2 transformation of abundance treatment/abundance NSC ratios (log2FC), averaged across all 6 donors. Samples were collected at 24h after initiation of the colonic incubations. Log 2 fold change (FC) was indicated in bold where statistical significance or consistent increase/decrease occurred.
- Fig. 4: shows the impact of the test products on species level OTUs as described for Table 1.
- Fig. 5: shows indicated tamarind extract's activities on Firmicute species as described for Table 1.
- Fig. 6: shows tamarind FE/PSF extracts also stimulate the indicated Firmicutes OTUs as described for Table 1.
- Fig. 7: shows that SE specifically and significantly increased OTUs related to the recently isolated species.
- Fig. 8: shows summary of prebiotic effects and metabolic modulation by tamarind extracts.

### Examples

### Example 1: Preparation of tamarind plant extracts

There is a growing understanding that the gut microbiome's composition and activity significantly influence cognitive function, particularly through SCFA levels and other metabolites. By selectively modulating the gut microbiota with tamarind extracts, the inventors aimed to identify plant extracts, and combinations, which enhance beneficial bacterial growth to support cognitive health benefits.

Using an in-vitro Systemic intestinal fermentation research (SIFR) model system based on donor stool samples, the inventors sought to define how tamarind extracts alter gut bacterial metabolite production and microbial composition (Fig. 1A and B). The flow chart in Fig. 1C summarises representative methods for obtaining botanical extracts from tamarind seed, and tamarind fruit, providing full seed extract (SE), full fruit extract (FE), and enriched fractions, including a fruit polysaccharide fraction (PSF), and fruit polysaccharide and polyphenol fraction (PPF) and subsequently submitted to the SIFR model system. Samples of tamarind extracts were first prepared using n-hexane as a solvent, followed by extraction with methanol, to isolate biologically active compounds.

To ensure compliance with food-grade regulatory standards in regions where n-hexane or methanol may not be approved for use in edible products, the organic solvent step using a non-polar solvent (e.g., for seed pre-processing) can instead employ ethyl acetate, a food-safe alternative, to selectively extract lipophilic components while avoiding hydrocarbon residues. Similarly, ethanol (e.g., pharmaceutical or food-grade ethanol) may replace methanol in extraction and fractionation steps, ensuring reduced toxicity risks and alignment with strict regulatory guidelines for ingestible products.

These substitutions yield extracts of comparable efficacy: ethyl acetate effectively removes seed lipid constituents, while ethanol-based maceration retains polysaccharides and polyphenols from the fruit or seed kernel without compromising downstream processing (e.g., Diaion HP-20 chromatography). The resulting tamarind extracts maintain their prebiotic potential in promoting bacteria like *Agathobaculum butyriciproducens* and *Coprococcus catus,* as validated by *in vitro* fermentation studies. These solvent adjustments ensure broad regulatory compliance (e.g., FDA, EU Food Safety), expanding the invention's applicability to food-grade or clinical formulations while preserving functional outcomes.

### Tamarind seeds

Tamarind seed powder, derived from the kernel from which the testa had been removed, (40 g, Balu Herbals Pvt Ltd, Plot No 78, Anmagal, Near Banjara Colony, Hayathnagar, Telangana 501505, India) was extracted for 24 h with 400 mL 100% n-hexane by maceration under stirring at room temperature. Another alkane or alkane mixture (petroleum ether) would also be appropriate, with less lipophilic solvents a lower yield of desired metabolites would be expected. The solvent was removed by filtration, and the solid residue was reextracted for 24 h with another 400 mL hexane. After the solvent was removed by filtration, the solid residue was extracted twice with 400 mL of methanol by maceration at room temperature for 24 h each time. The filtrates were combined and evaporated to dryness to yield 1.11 g of dry methanol seed extract.

Representative step-by-step protocol (Fig. 1C):
1. Extraction of seed powder by maceration with 400 mL n-hexane for 24 h
2. Filtration and re-extraction of the solid residue with 400 mL n-hexane for 24 h
3. Filtration and extraction of the solid residue with 400 L methanol for 24 h
4. Filtration to provide filtrate 1 and re-extraction of the solid residue with 400 mL methanol for 24 h
5. Filtration to provide filtrate 2.
6. Evaporation to dryness of the combined filtrates 1 and 2 to provide the dry methanol seed extract

### Tamarind fruit pulp

Tamarind fruit powder (40 g, Ae Naturals, 1253, Amazing Villa, 8th Cross, Gokle Road, , opp. New Horizon Public School, 3rd Stage BEML Layout, Rajarajeshwari Nagar, Bengaluru, Karnataka 560098, India) was extracted twice with 400 mL of methanol by maceration at room temperature for 24 h each time. After filtration, both extracts were combined and evaporated to dryness. The dry methanol extract of the fruits (except 210 mg which were kept) was dissolved in 500 mL H2O and passed through a polystyrene/divinylbenzene resin Diaion HP-20 column (2.5 x 20 cm, Supelco) in a reverse phase chromatography process. The column was then washed with ca. 800 mL H2O and then eluted with methanol (ca. 400 mL). 250 mL of the water-eluted fraction were lyophilized to yield 3.23 g of polysaccharide fraction. The MeOH-eluted fraction was evaporated to dryness to yield 247 mg of polyphenolic fraction.

To obtain an additional amount of fruit methanol extract, 20 g of fruit powder were extracted with 200 mL methanol for 24 h by maceration at r. t.. After filtration, evaporation of the filtrate under reduced pressure yielded 5.5 g of dry methanol extract.

Representative step-by-step protocol (see Figures for table and flowchart):
1. Extraction of fruit powder with 400 mL methanol for 24 h
2. Filtration to provide filtrate 1 and re-extraction of the solid residue with 400 mL methanol for 24 h
3. Filtration to provide filtrate 2
4. Evaporation to dryness of combined filtrates 1 and 2 to provide the dry methanol fruit extract
5. Dissolution of the dry methanol fruit extract in 500 mL water
6. Passing through a Diaion HP-20 column and further elution of the column with 800 mL water
7. Lyophilisation of the water eluate to provide the dry polysaccharide fraction
8. Elution of the HP-20 column with 400 mL methanol
9. Evaporation of the methanol eluate to provide the dry polyphenolic fraction

### In-vitro assessment of tamarind extract on donor stool samples:

The SIFR (Systemic Intestinal Fermentation Research) methodology was applied by Cryptobiotix SA, Technologiepark-Zwijnaarde 82, 9052 Gent, Belgium).

An ex vivo SIFR study was implemented, simulating the colonic fermentation of test products by the gut microbiota-derived from human adults (n = 6). The simulation parameters were as follows:
- Study arms = 5:
   - No substrate control (NSC) = background medium + microbiota (no product)
   - 4 test products
- Time points = 0h (only NSC), 24h

Timeline and analyses:
Key fermentation parameters: pH, gas production, SCFA (acetate, propionate, butyrate and valerate), bCFA (isobutyrate, isovalerate) and total SCFA at 0h (only NSC) and 24h (all test arms)
Microbial composition analysis (quantitative 16S rRNA gene profiling) at 0h (NSC) and 24h (all test arms)

Four different tamarind extracts, i.e., seed extract, full extract of the fruits, polysaccharide and polyphenolic fraction were tested against a no-substrate control. The polysaccharide fraction, the seed extract and full extract were tested at 5 g/L (Table 1) (= standard dose to test fibres in the SIFR^{®} technology). Since the polyphenol content of the polyphenolic fraction was 50%, the polyphenolic fraction was dosed at 0.8 g/L to reach a final concentration of 0.4 g polyphenols/L (= standard dose to test polyphenols in the SIFR^{®} technology; enables to see modulatory effects while avoiding strong antimicrobial effects).

Acetate, propionate, butyrate, valerate and branched chain fatty acids, (bCFA) (sum of isobutyrate, isovalerate) were determined via a GC-FID approach. Further, pH and gas production were also measured as key markers for microbial metabolic activity.

### Example 2: Tamarind extracts modulate key fermentation parameters and abundance of beneficial commensal organisms

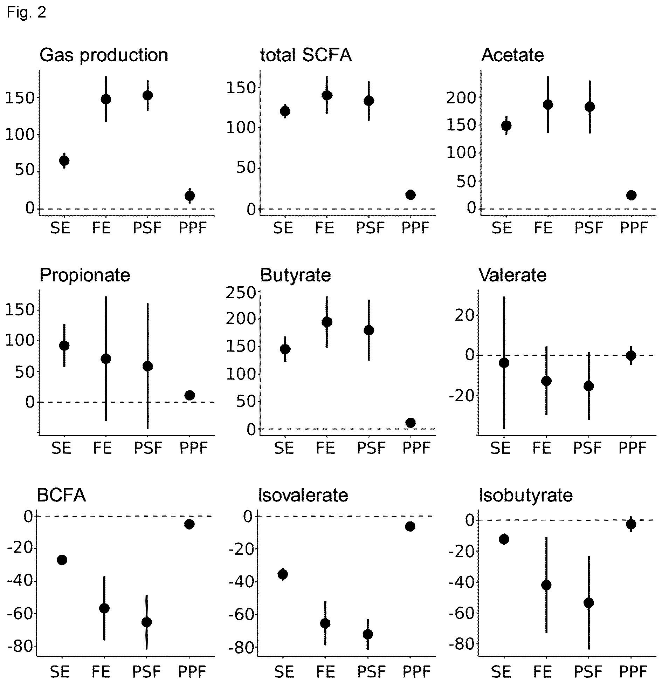

The seed extract (SE) and full fruit extract/polyphenolic fraction (FE/PFS) increased acetate, propionate, butyrate, and total SCFA levels while decreasing isobutyrate and isovalerate (bSCFAs). Three extracts (SE, FE, PSF) induce SCFAs to a level where beneficial health effects are expected, based on piglet studies (Fig. 2) (Boudry, 2010).

The SE extract induces remarkably low gas production while still driving robust induction of butyrate and SCFA. The SE extract shows remarkably little variation across the six human donors in inducing SCFAs, suggesting this substrate may be used broadly to achieve metabolic impact in combination with commensal species that may differ across subjects, rather than targeting a narrower range of less commonly present commensals.

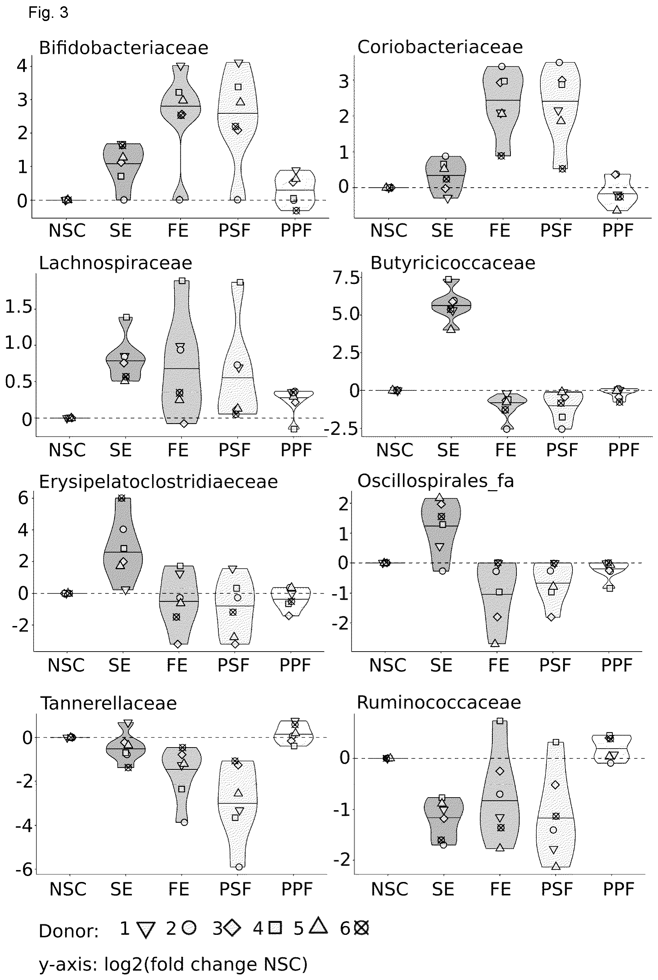

SE and PSF strongly promoted *Bifidobacteriaceae, Coriobacteriaceae* and *Lachnospiraceae,* at the expense of several *Bacteroidota* families (mainly *Prevotellaceae* and *Tannerellaceae*), *Firmicutes* families (e.g. *Butyricicoccaceae, Monoglobaceae, Oscillospiraceae, Peptostreptococcaceae, Ruminococcaceae*), *Desulfovibrionaceae, Sutterellaceae* and *Akkermansiaceae*). Further, SE, besides also boosting *Bifidobacteriaceae* and *Lachnospiraceae,* specifically stimulated the *Firmicutes* families *Butyricicoccaceae, Erysipelatoclostridiaceae, Oscillospirales.* While the effects of PPF were milder compared to the other test products, PPF significantly increased *Lachnospiraceae* and also tended to increase *Bifidobacteriaceae, Tannerellaceae* and *Ruminococcaceae* (Fig. 3, Table 1).

**Table 1. shows the impact of the test products on species level OTUs that were significantly (p adjusted < 0.20, indicated by *) or consistently affected (indicated by $) by any of the treatments, expressed as log2 transformation of abundance treatment/abundance NSC ratios (log2FC), averaged across all 6 donors. Samples were collected at 24h after initiation of the colonic incubations. Log 2 fold change (FC) was indicated in bold where statistical significance or consistent increase/decrease occurred.**

| **Name** | **SE** | **FE** | **PSF** | **PPF** |
|---|---|---|---|---|
| *Bifidobacterium adolescentis** | 1.11 | 2.68 | 2.6 | 0.28 |
| *Bifidobacterium longum** | 0.7 | 1.01 | 0.36 | 0.27 |
| *Collinsella aerofaciens** | 0.34 | 2.42 | 2.35 | -0.1 |
| *Bacteroides ovatus** | -0.72 | 0.6 | 0.89 | -0.3 |
| *Bacteroides faecis** | -0.56 | -0.14 | -0.25 | -0.2 |
| *Bacteroides uniformis** | -0.09 | -1.68 | -2.22 | -0.2 |
| *Bacteroides stercoris** | -1.04 | -0.46 | -1.58 | -0.05 |
| *Bacteroides massiliensis** | -0.33 | -1.44 | -1.76 | -0.12 |
| *Bacteroides cellulosilyticus$* | -0.11 | 0.03 | 0.53 | -0.39 |
| *Phocaeicola vulgatus** | 0.03 | -0.89 | -2.19 | -0.19 |
| *Segatella copri#* | -1.75 | -3.44 | -4.05 | -0.08 |
| *Parabacteroides merdae** | -0.36 | -2.1 | -3.17 | -0.24 |
| *Parabacteroides distasonis** | -0.46 | -1.49 | -3.56 | 0.59 |
| *Agathobaculum butyriciproducens*#* | 5.5 | -0.89 | -0.88 | -0.31 |
| *Ruminiclostridium cellulolyticum (95.5%)** | -0.34 | -1.21 | -1.29 | -0.2 |
| *Faecalibacillus intestinalis*#* | 2.96 | -0.64 | -1.36 | -0.36 |
| *Blautia faecis** | 0.98 | 1.23 | 1.35 | 0.79 |
| *Fusicatenibacter saccharivorans ** | 1.59 | 0.39 | -0.2 | 0.6 |
| *Blautia obeum** | 1.29 | -0.64 | -1.44 | 0.29 |
| *Dorea formicigenerans** | -2.46 | 0.19 | 0.12 | -0.09 |
| *Clostridium fessum** | 0.41 | -0.65 | -1.03 | 0.05 |
| *Oliverpabstia intestinalis** | 1.13 | 1.69 | 1.58 | 0.77 |
| *Mediterraneibacter faecis#* | 1.25 | 3.23 | 2.87 | 0.13 |
| *Anaerobutyricum hallii** | 0.06 | 2.18 | 2.09 | 0.36 |
| *butyrate-producing bacterium SS3*/*4** | 0.15 | -0.91 | -1 | 0.97 |
| *Eubacterium ramulus** | -0.69 | -1.28 | -2.02 | -0.14 |
| *Blautia fusiformis** | 2.43 | 0.47 | 0.13 | -0.31 |
| *Coprococcus eutactus** | -0.52 | -1.25 | -1.21 | -0.14 |
| *Roseburia inulinivorans** | -1.59 | -0.46 | -1.05 | -0.08 |
| *Lachnoclostridium pacaense (96.3%)** | 1.19 | 0.87 | 1 | -0.07 |
| *Clostridium clostridioforme_bolteae** | 0.89 | 0.57 | 0.68 | 0.47 |
| *Coprococcus catus*#* | 3.72 | -1.02 | -0.9 | -0.04 |
| *Anaerotruncus lactatifermentans*#* | -0.68 | -3.62 | -4.25 | -0.65 |
| *Bacteroides galacturonicus** | -2 | -1.63 | -2.05 | -0.12 |
| *Brotolimicola acetigigens** | 0.05 | -1.21 | -1.68 | -0.43 |
| *Eubacterium eligens** | -0.04 | -0.88 | -1.18 | 0.16 |
| *Anaerostipes hadrus** | 3.3 | -0.51 | -0.51 | -0.08 |
| *Ruminococcus torques** | -1.82 | -0.66 | -1.2 | -0.17 |
| *Gallinestinimicrobium propionicum** | 0.2 | -1.19 | -1.23 | 0.21 |
| *Coprococcus comes** | -1.69 | 1.46 | 1.64 | 0.07 |
| *Hominilimicola faborformis** | -0.54 | -2.47 | -3.56 | -0.54 |
| *Oscillibacter sp** | -1.08 | -1.48 | -1.66 | -0.46 |
| *Brotocaccenecus cirricatena** | -0.36 | -1.31 | -1.7 | -0.05 |
| *Hominicoprocela fusiformis** | -1.5 | -3.1 | -3.08 | -0.72 |
| *Oscillospiraceae sp** | 2.1 | -0.78 | -1.13 | -0.25 |
| *Romboutsia ilealis$* | -0.12 | -0.92 | -0.43 | -0.25 |
| *Faecalibacterium prausnitzii** | -1.7 | -0.75 | -1.21 | -0.25 |
| *Gemmiger formicilis** | -1.16 | -1.05 | -1.22 | -0.22 |
| *Escherichia coli** | -0.49 | -0.3 | 0.32 | -0.23 |

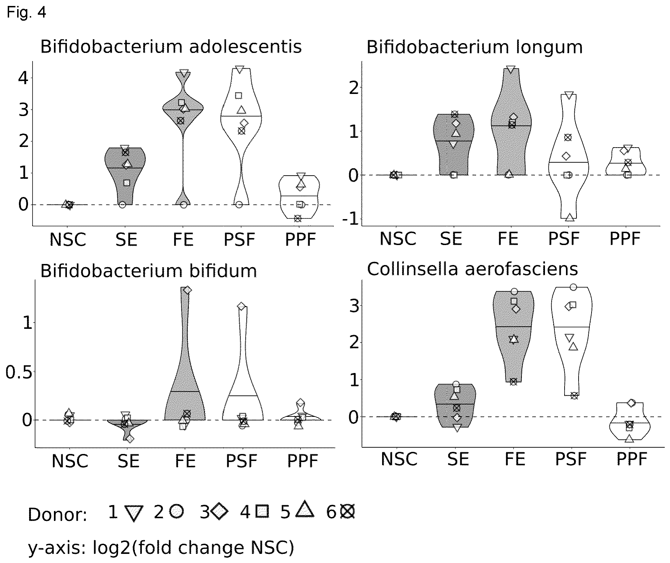

At the species level, further modulatory effects could be observed (Fig. 4). Tamarind FE/PSF, displayed strong bifidogenic effects, as evidenced by the strong increase in OTUs related to acetate-producing *Bifidobacterium adolescentis, B. bifidum* (one donor) with particularly FE also increasing *B*. *longum.* Further, SE and PPF also respectively strongly and mildly increased *B*. *adolescentis*/*B. longum.* Besides acetate, producing *Bifidobacterium* spp. also produced lactate which are both important cross-feeding metabolites used by butyrate producers. This aligned with the positive correlations between *Bifidobacterium adolescentis* and acetate/butyrate production, particularly for FE/PSF. The strong stimulatory effects of FE/PSF on *Actinobacteriota* was further evidenced by their stimulation of *Collinsella aerofaciens* (OTU5). In a previous study, a *C*. *aerofaciens* subspecies was demonstrated to be able to biosynthesize butyrate. Further, *Collinsella* species also produce ursodeoxycholate which was previously reported to suppress pro-inflammatory cytokines and have antioxidant and anti-apoptotic effects (Hirayama, 2021).

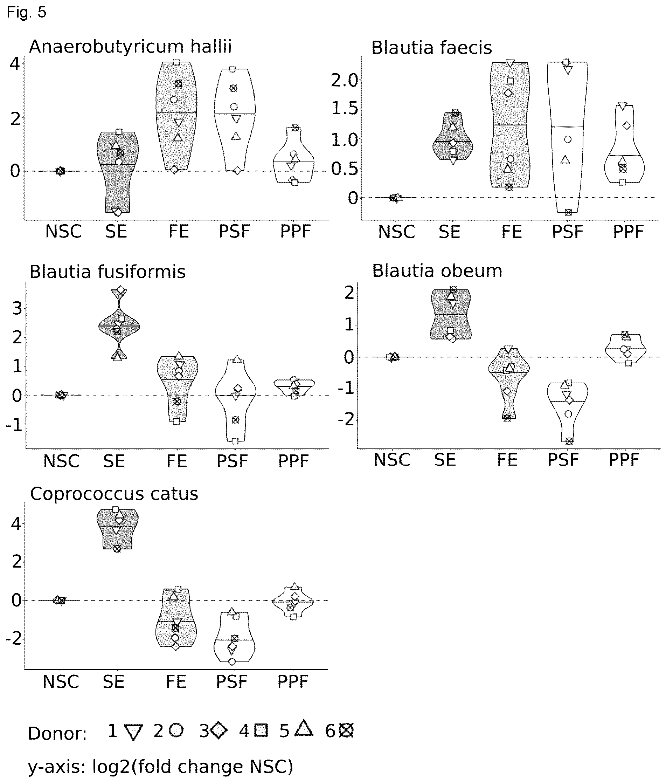

The extracts modified species with known capability of producin both propionate and butyrate: FE/PSF increased OTUs related to *Anaerobutyricum hallii* and *Blautia faecis.* SE significantly stimulated OTUs related to *Blautia faecis, Blautia fusiformis, Blautia obeum and Coprococcus catus* (Fig. 5).

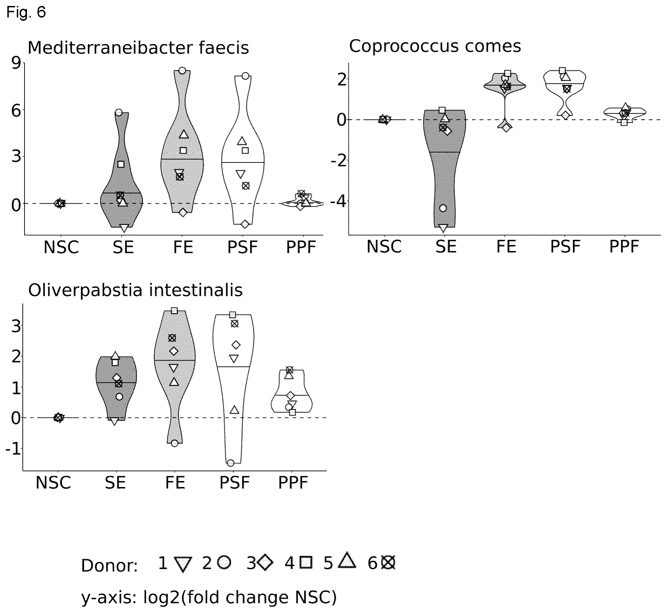

PPF significantly increased *Blautia faecis* and tended to increase the OTU related to *B. fusiformis* and *B. obeum.* FE/PSF significantly increased OTUs related to *Mediterraneibacter faecis,* the prominent butyrate producer *Coprococcus comes* as well as *Oliverpabstia intestinalis* (also increased for PPF) which is a predicted butyrate producer (Fig. 6).

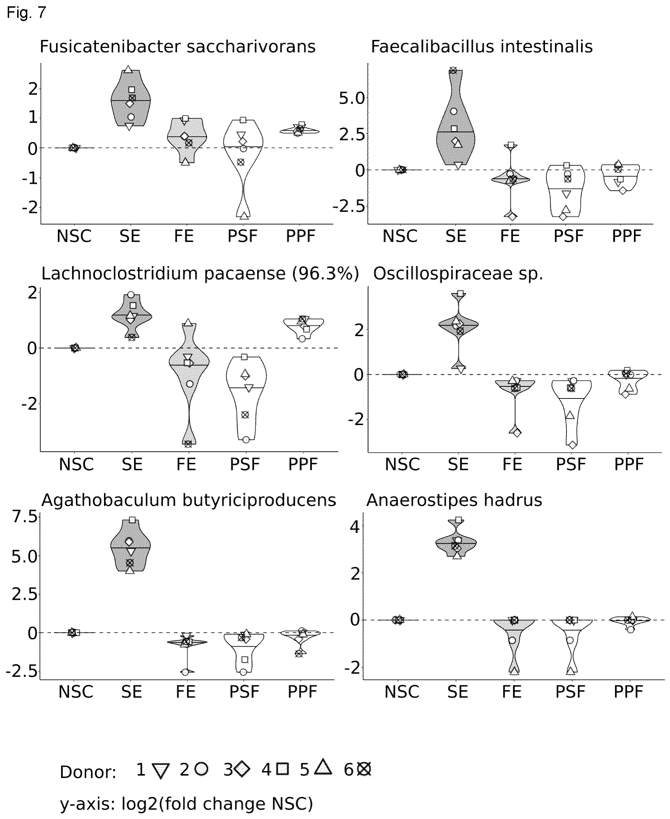

SE specifically and significantly increased OTUs related to the recently isolated species *Fusicatenibacter saccharivorans* (also increased for PPF), *Faecalibacillus intestinalis* and the butyrate producers *Oliverpabstia intestinalis, Lachnoclostridium pacaense* (also increased for PPF), *Oscillospiraceae sp.* and especially *Anaerostipes hadrus* (*~* 10-fold) and *Agathobaculum butyriciproducens* (*~* 45-fold) (Fig 7).

### Example 3: Specific, robust and strong induction of neuromodulating species

The metabolic fermentation parameter changes correlated with specific beneficial bacteria stimulation (Fig 3-8, Table 1), including *B. adolescentis*/*longum, Blautia spp., C.catus, Anaostipes, A. butyriciproducens, Oliverpens,* and *P. distasonis,* which are known to have positive effects on cognitive function. The SE fraction strongly stimulates bacteria involved in neuroinflammatory processes, such as *A. butyriciproducens* and *C. catus.*

*A. butyriciproducens* is a butyrate-producing bacterium that plays a crucial role in maintaining a balanced gut microbiota, regulating the host immune response, and enhancing intestinal mucosal barrier function. Besides health benefits associated with butyrate, the neuroprotective effects of *A. butyriciproducens* are particularly of interest. In murine models, *A. butyriciproducens* was recently shown to improve cognitive impairment related to both aging and Alzheimer's disease. Between 96 and 99% of Europeans have it in their gut in detectable amounts, suggesting SE is a strong candidate for a prebiotic compatible with a broad swathe of the population according to analyses by Unseenbio (https://my.unseenbio.com). *A. butyriciproducens* growth is stimulated by fermented foods, and strongly repressed by sweetened softdrinks and other unhealthy foods (Boktor, 2023). By having an extract that stimulates *A. butyriciproducens* growth in the gut, extracts from seed according to the invention can offset and compensate the deleterious effects of such foods as documented in Parkinson's patients.

The SE extract enhanced abundance of another a highly beneficial species *C.s catus* studied in the context of inflammation (see for example, CN104546951). The *Coprococcus* genus has also been associated with attenuated symptoms of cognitive impairment in neurological disorders (Notting F. et al. (2023) Gut Microbiome: 4:e16), and to attenuate stress-induced depression-like changes in murine studies (Xu et al. (2024) J. Affective Disorders 356:171). These effects are predicted to be robustly relevant to the population, as extracts achieved induction achieved in all 6 human stool samples tested (100% success rate). The effect was also rapid, as evidenced by a strong, 15-fold upregulation of targeted bacteria within just 24 hours.

The compositions of the invention target specific microbial populations within the gut, promoting SCFA levels associated with cognitive function enhancements. Synbiotic combinations can leverage these prebiotic effects to enhance probiotic survival and efficacy, while postbiotics derived from controlled fermentation provide stable therapeutic metabolites without the production complexity and regulation hurdles presented by live bacteria.

### Example 4: Rational design of pharmaceutical compositions comprising tamarind extracts

The results of the in vitro system suggest novel compositions and methods for modulating the human gut microbiome using tamarind extracts to achieve specific cognitive benefits, particularly by enhancing SCFA production. The determined that tamarind seed extract (SE), full fruit extract/polyphenolic fraction (FE/PFS) are plausible active agents capable of altering microbial metabolism and composition. When administered alone or in combination with probiotics, these extracts could enhance beneficial bacterial growth and metabolite production associated with anti-inflammatory properties and cognitive health benefits (Fig. 8).

Tamarind extracts can be provided for use alone, or in synbiotic combinations with bacterial strains. Examples of suitable strains that may be enhanced by the extracts of the present invention include, for instance, those deposited with the Leibniz Institute DSMZ-German Collection of Microorganisms and Cell Cultures or the American Type Culture Collection (ATCC) under the following accession numbers: DSM20083 (B. adolescentis), DSM12568 (Blautia spp.), DSM100391 (A. butyriciproducens), ATCC27761 (C. catus).

### Tamarind seed extracts and probiotic combinations

In certain embodiments, the pharmaceutical composition comprises a tamarind seed extract, particularly a tamarind seed extract obtainable by a method according to Example 1, Figure 1C. Such SE provide strong induction of SCFA-producing metabolic pathways, and have the benefit of low-gas properties favourable in prebiotics.

In particular embodiments, the SE fraction is prepared according to the flow chart illustrating the process in Figure 1C. Optionally, the powder is added to an aqueous solution to provide a pharmaceutical composition comprising a tamarind seed extract according to the invention.

The findings in the Examples demonstrate the utility of prebiotic compositions comprising a tamarind seed extract, for use in increasing the relative abundance in the intestine of recipient of a endogenous commensal strain as shown in the targeted enhancement in Fig. 8. Optionally, a probiotic formulations can be formed by combining the relevant fruit pulp extract with live bacterial preparations of target strains.

In particular embodiments, the composition comprises a tamarind seed extract, and a live bacteria of a probiotic commensal strain, particularly a commensal strain selected from a species in the genus *Blautia,* particularly *B. faecis, B. fusiformis, B. obeum.* In other embodiments, the composition comprises a tamarind seed extract, and a live bacteria of a probiotic commensal strain of the species *A. butyriciproducens,* and/or *C. catus.*

### Tamarind fruit extracts and probiotic combinations

In certain embodiments, the pharmaceutical composition comprises a tamarind fruit pulp extract, particularly a tamarind full fruit pulp extract (FE) obtainable by a method according to Example 1, Figure 1C. Such FE provide strong induction of SCFA-producing metabolic pathways, and enhance the abundance of beneficial strains (Fig. 8). FE are favourable prebiotics targeting these strains.

In some embodiments, the tamarind fruit extract is an unfractionated fruit pulp extract. In other embodiments, the tamarind fruit extract is a fraction with enhanced polysaccharide content obtained from a tamarind fruit according to the method Example 1, Figure 1C. In particular embodiments, the polysaccharide content of the fraction is more than 50%.

A first useful composition comprises a fruit pulp extract and/or a polyphenolic and polysaccharide fruit pulp fraction (FE/PFS) or a combination thereof, each containing specific polyphenol and polysaccharide content as detailed in Figure 1.

In particular embodiments, a FE/PFS fraction is obtained through water extraction, with an additional step involving methanol precipitation to enrich polyphenolic compounds (>50%) while retaining at least 40% polysaccharide content.

The findings in the Examples demonstrate the utility of prebiotic compositions comprising a tamarind fruit pulp extract, for use in increasing the relative abundance in the intestine of recipient of a endogenous commensal strain as shown in the targeted enhancement achieved by separated fractions of tamarind fruit pulp in Fig. 8. Optionally, a probiotic formulations can be formed by combining the relevant fruit pulp extract with live bacterial preparations of target strains.

### Combination of tamarind fruit extracts and tamarind seed extracts, optionally in combination with probiotic strains

The inventors found that all studied fruit extracts promoted the abundance of members of *Bifidobacterium.* Such extracts are relevant as prebiotic or post biotic compositions to be applied to enhance the abundance of commonly commercially available probiotics species that are often provided as dietary supplements (e.g., *Bifidobacteria*) providing probiotic growth.

Specific tamarind plant extracts had additional effects, for example SE promotes endogenous strains which are present in >98% of European intestinal microbiome samples, such as *Agathobaculum* species.

In certain embodiments, the pharmaceutical composition comprises both a tamarind seed extract, and a tamarind fruit pulp extract, Optionally, the composition may further comprise a probiotic commensal strain, particularly a probiotic commensal strain selected from those listed in Table 1. This provides a combination therapy that enhances abundance of endogenous strains, and at the same time may support engraftment of a probiotic composition comprising live bacteria (Fig. 9).

### Administration and dosing

Synbiotic combinations are prepared by combining tamarind extracts (SE or FE/PFS) with bacterial strains. In particular embodiments, the live bacteria in the composition has a concentration of 10^7 CFU/g for each strain.

One recommended daily dose is 200mg-2000mg of the tamarind extract(s), alone or in combination with probiotics, to significantly increase beneficial bacterial populations and corresponding SCFA production.

The compositions may be administered orally to enhance cognitive function, particularly by increasing SCFA levels associated with improved memory and learning capabilities. By targeting specific microbial populations, these compositions address cognitive decline, enhance SCFA levels known for their anti-inflammatory properties, and provide stable postbiotic formulations without live bacteria. The synbiotics offer synergistic effects through enhanced probiotic survival and efficacy. These advancements fulfil a critical need in modern health management by offering precise microbial modulation for cognitive benefits.

### Methods

Metagenomics: Upon DNA extraction, library preparation and sequencing were performed on an Illumina MiSeq platform with v3 chemistry. The 16S rRNA gene V3-V4 hypervariable regions were amplified using primers 341F and 785Rmod. Results were analysed at different taxonomic levels (phylum, family and OTU level). For taxonomic analysis, the proportional data derived from sequencing (%) were corrected for the total amount of cells present in each sample (detected via flow cytometry), allowing to obtain more representative insights in the impact of interventions on the gut microbiota27.

Data Analysis: For exploratory evaluation of the obtained results, a series of principle component analysis (PCA) was performed. The two principal components with the largest eigenvalues were plotted. For the statistical evaluation of the treatment effects on key fermentation parameters, cell counts, microbial diversity (4 indices) and microbial composition (phylum level) across 6 different donors, a repeated measures ANOVA analysis was performed (~ based on paired t-testing, thus accounting for fact that values are compared between samples of a given donor). The statistical significance of the potential treatment effects was determined via Benjamini-Hochberg post hoc testing28. The latter involves that a correction for multiple comparisons was implemented where p-values were adjusted by multiplying them with the total number of comparisons divided by the rank of each original p-value (across all p-values). In this specific case, 6 comparisons were considered (= 4 comparisons (NSC vs. 4treatments), 2 additional comparisons of PPF/PSF vs FE) (Figure 3). In practice, this means that while the largest obtained p-value remained uncorrected (i.e., multiplied 1), the lowest p-value was multiplied with 6, thus strongly decreasing the chance of type 1 errors (i.e. false positives). In addition, the resulting series of adjusted p-values was rendered non-decreasing (i.e., when an adjusted p-value was higher than any of the subsequent adjusted p-values, its value was equalled to this lowest value). This generates a false discovery rate threshold allowing to estimate and control the chance of type 1 errors (i.e., false positives).

Statistics: For the statistical evaluation of the treatment effects on microbial composition (family and OTU level), the Benjamini-Hochberg correction was applied within each comparison (NSC vs. 4 test products), given the large number of features analysed. As a remark, for statistical analysis of the quantitative 16S rRNA gene profiling, a value below the limit of quantification (LOQ) was equalled to the LOQ. Then, statistics was performed based on log- transformation of the absolute values (to render the data normally distributed). To establish an overall LOQ, first, 1 read was divided by the total amount of reads in each sample, followed by multiplication with the bacterial cell count detected via flow cytometry. This allowed to obtain a LOQ for each sample individually. Then, the highest LOQ was used as overall LOQ of the entire dataset. To illustrate the most representative change for each taxon, fold changes against the NSC were calculated for individual donors. Subsequently, log2-transformation was performed on the geometric mean of these fold changes. Geometric mean was used since it is more accurate and representative than the often-used arithmetic mean, especially for positively skewed data which is often the case for microbial composition data (due to interpersonal variances). Further, regularized Canonical Correlation Analysis (rCCA) was performed to highlight correlations between metabolites and compositional data (at OTU level). Regarding compositional data, log- transformed, absolute phylogenetic data was used as input. rCCA was executed using the mixOmics package with the shrinkage method for estimation of penalisation parameters in R (https://www.r-project.org/).
* (0.1 < p adjusted < 0.2), ** (0.05 < p adjusted < 0.1) or *** (p adjusted < 0.05) for treatment vs NSC
$ (0.1 < p adjusted < 0.2), $$ (0.05 < p adjusted < 0.1) or $$$ (p adjusted < 0.05) for PSF/PPF vs FE

### REFERENCES

All scientific publications and patent documents cited in the present specification are incorporated by reference herein.
Boudry et al. 2013 J. Nutr.143(8): 1225.
Sambrook et al., Molecular Cloning: A Laboratory Manual, 4th ed. (2012) Cold Spring Harbor Laboratory Press, Cold Spring Harbor, N.Y.
Ausubel et al., Short Protocols in Molecular Biology (2002) 5th Ed, John Wiley & Sons, Inc.
Remington: the Science and Practice of Pharmacy, ISBN 0857110624
Boktor et al. 2023 Mov. Disord. 38(3):399 (PMID36691982)
CN104546951
Notting F. et al. 2023 Gut Microbiome: 4:e16
Xu et al. 2024 J. Affective Disorders 356:171
Hirayama et al. 2021 PLoS ONE 16(11): e0260451.
Walker HK, Hall WD, Hurst JW, Clinical Methods: The History, Physical, and Laboratory Examinations. 3rd edition. (1990).

## Claims

1. A composition comprising a tamarind fruit extract for use in:
i. increasing the relative abundance of a beneficial bacteria in the intestine of a patient; and/or
ii. increasing the presence of acetate, propionate, and/or butyrate in the intestine of a patient;
particularly wherein the composition comprises a tamarind seed extract, and/or a tamarind fruit pulp extract.

2. The composition for use according to claim 1, wherein the beneficial bacteria is a species selected from *Collinsella aerofaciens, Anaerobutyricum hallii, Blautia faecis, Mediterraneibacter faecis, Oliverpabstia intestinalis, Coprococcus comes, Parabacteroides distasonis, Butyrate-producing bacterium SS3*/*4, Faecalibacterium prausnitzii, Fusicatenibacter saccharivorans, Lachnoclostridium pacaense, Coprococcus catus, Blautia fusiformis, Blautia obeum, Anaerostipes hadrus, Agathobaculum butyriciproducens, Faecalibacillus intestinalis, Fusicatenibacter saccarivorans, Bifidobacterium adolescentis, Bifidobacterium bifidum,* and *Bifidobacterium longum.*

3. A symbiotic composition comprising a tamarind fruit extract and a live bacterium selected from a *Collinsella aerofaciens, Anaerobutyricum hallii, Blautia faecis, Mediterraneibacter faecis, Oliverpabstia intestinalis, Coprococcus comes, Parabacteroides distasonis, Butyrate-producing bacterium SS3*/*4, Faecalibacterium prausnitzii, Fusicatenibacter saccharivorans, Lachnoclostridium pacaense, Coprococcus catus, Blautia fusiformis, Blautia obeum, Anaerostipes hadrus, Agathobaculum butyriciproducens, Faecalibacillus intestinalis, Fusicatenibacter saccarivorans* and a species in the genus *Bifidobacterium,* particularly wherein the composition comprises a tamarind seed extract, and/or a tamarind fruit pulp extract.

4. The composition according to any one of claims 1 to 3, for use in improving symptoms of cognitive impairment in a patient suffering therefrom, particularly wherein the composition comprises a tamarind seed extract, and/or a tamarind fruit pulp extract.

5. The composition for use according to any of the preceding claims, wherein the patient has a disease or disorder that causes or is associated with neuroinflammation.

6. The composition for use according to any of the preceding claims, for use in improving symptoms of a patient diagnosed with depression, Alzheimer's disease, Parkinson's disease, age-related cognitive impairment, or an autism spectrum disorder.

7. A non-therapeutic method of improving the cognition, mood and/or quality of life (QoL) in a subject as measured using a clinical scoring method, wherein the method comprises orally administering a composition comprising a tamarind fruit extract to the subject, particularly wherein the composition comprises a tamarind seed extract, and/or a tamarind fruit pulp extract.

8. The non-therapeutic method according to claim 7, wherein the subject is a healthy individual.

9. The non-therapeutic method according to claim 7 or 8, wherein the subject is an elderly individual, particularly wherein the elderly individual is at least 55, at least 60, at least 65, at least 70, at least 75 or at least 80 years old.

10. The non-therapeutic method according to any one of claims 7 to 9, wherein the improved is associated with increasing the relative abundance of a beneficial bacteria in the intestine of a patient; and/or increasing the presence of acetate, propionate, and/or butyrate in the intestine of the subject.

11. The composition for use, the symbiotic composition or the non-therapeutic method according to any of the preceding claims, wherein the composition comprises, or consists of a tamarind seed extract, and/or a tamarind fruit pulp extract.

12. The composition for use, the symbiotic composition or the non-therapeutic method according to any one of the preceding claims, wherein the composition comprises, or consists of tamarind fruit pulp extract comprising:
i) a tamarind fruit pulp polyphenolic fraction comprising at least 40% polysaccharide compounds, and at least 50% polyphenol compounds; and/or
ii) a tamarind fruit pulp polysaccharide fraction comprising at least 50% polysaccharide compounds.

13. The composition for use, the symbiotic composition or the non-therapeutic method according to any one of the preceding claims, wherein the tamarind fruit extract comprises, or consists of tamarind seed extract or tamarind seed kernel extract.

14. The composition for use, the symbiotic composition or the non-therapeutic method according to any one of the preceding claims, wherein the tamarind seed extract and/or tamarind pulp extract have been obtained by a method comprising the following steps:
a. providing tamarind plant matter selected from a tamarind seed preparation, or tamarind fruit pulp preparation;
b. in an alcohol solvent step, soaking the tamarind plant matter in an alcohol, providing an alcoholic extract;
c. separating insoluble plant matter from the alcoholic extract, and removing the alcohol from the alcoholic extract to provide a dried tamarind seed extract, or a dried tamarind fruit pulp extract.

15. The composition for use, the symbiotic composition or the non-therapeutic method according to claim 14, wherein the method further comprises
d. dissolving the dried tamarind fruit pulp extract obtained in claim 8 in an aqueous solution; and
e. in a fractionation step, isolating from the aqueous solution comprising the dried tamarind fruit pulp extract:
• a fraction comprising at least 50% polysaccharide compounds, and/or
• a fraction comprising a tamarind fruit polyphenolic fraction comprising at least 40% polysaccharide compounds, and at least 50% polyphenol compounds.

16. The composition for use, the symbiotic composition or the non-therapeutic method according to 15, wherein the fractionation step is performed by means of liquid chromatography (LC), particularly by means of reversed-phase liquid chromatography (RPLC).

17. The composition for use, the symbiotic composition or the non-therapeutic method according to any of the claims 14 to 16, wherein the alcohol is ethanol, or methanol.

18. The composition for use, the symbiotic composition or the non-therapeutic method according to any one of claim 14 to 17, particularly wherein the tamarind plant matter comprises, or essentially consists of tamarind seed, wherein prior to the solvent step, the method further comprises,
a. in a solvent step, soaking the tamarind plant matter with an organic solvent providing a solvent extract,
b. separating soluble residue from the solvent extract, and removing the organic solvent from the solid residue,
and wherein in the alcohol solvent step as specified in claim 8, the solid residue obtained in step b. is soaked in the alcohol to provide the alcoholic extract.

19. The composition for use, the symbiotic composition or the non-therapeutic method according to claim 18, wherein the organic solvent is ethyl acetate, or n-hexane.

20. The composition for use, the symbiotic composition or the non-therapeutic method according to any one of claims 14 to 19, wherein the duration of the soaking in the solvent step and/or the non-polar solvent step is at least 24 hours.
